# EUROPÄISCHE PATENTSCHRIFT

(11) **EP 2 931 333 B1**
(45) Veröffentlichungstag und Bekanntmachung des Hinweises auf die Patenterteilung: **28.03.2018**
(21) Anmeldenummer: 13798932.3
(22) Anmeldetag: 19.11.2013
(51) Int. Cl.: A61M 1/36

(54) **VORRICHTUNG UND VERFAHREN ZUM ÜBERWACHEN EINES EXTRAKORPORALEN BLUTKREISLAUFS ZUR ERKENNUNG VON LUFTBLASEN**
DEVICE AND METHOD FOR MONITORING AN EXTRACORPOREAL BLOOD CIRCULATION FOR DETECTION OF AIR BUBBLES
DISPOSITIF ET PROCÉDÉ DE SURVEILLANCE D'UN CIRCUIT SANGUIN EXTRACORPOREL POUR DÉTECTER DES BULLES D'AIR

(30) Priorität: 13.12.2012 DE 102012024341; 13.12.2012 US 201261736672 P
(43) Veröffentlichungstag der Anmeldung: 21.10.2015
(73) Patentinhaber: Fresenius Medical Care Deutschland GmbH, 61352 Bad Homburg v.d.H (DE)
(72) Erfinder: GAGEL, Alfred, 96123 Litzendorf (DE); WIESEN, Gerhard, 61352 Bad Homburg v.d.H. (DE)
(74) Vertreter: Oppermann, Frank
(86) Internationale Anmeldenummer: PCT/EP2013/003478
(87) Internationale Veröffentlichungsnummer: WO 2014/090370

(56) Entgegenhaltungen:
- EP-A2- 1 260 238
- US-A1- 2002 134 134
- US-A1- 2007 062 861
- US-A1- 2008 171 960
- US-A1- 2010 274 172

## Beschreibung

Die Erfindung betrifft eine Vorrichtung zum Überwachen eines extrakorporalen Blutkreislaufs einer extrakorporalen Blutbehandlungsvorrichtung zur Erkennung von Luftblasen in dem Blut, das in dem extrakorporalen Kreislauf von einer Blutpumpe gefördert wird. Darüber hinaus betrifft die Erfindung ein Verfahren zum Überwachen eines extrakorporalen Blutkreislaufs zur Erkennung von Luftblasen sowie eine Vorrichtung zur extrakorporalen Blutbehandlung mit einer Vorrichtung zur Überwachung des extrakorporalen Blutkreislaufs.

Es sind verschiedene Verfahren zur extrakorporalen Blutbehandlung bekannt, bei denen das Blut des Patienten in einem extrakorporalen Blutkreislauf durch eine Blutbehandlungseinheit strömt. Eine der wesentlichen Komplikationen bei der extrakorporalen Blutbehandlung, beispielsweise Hämodialyse oder Hämofiltration, stellt die Möglichkeit des Eindringens von Luft in den extrakorporalen Blutkreislauf dar.

Zum Abscheiden mitgeführter Luftblasen aus Blut dienen die bekannten Tropfkammern, die im venösen Zweig des extrakorporalen Blutkreislaufs stromab der Blutbehandlungseinheit angeordnet werden. Die bekannten Tropfkammern fangen die Luftblasen mit hoher Sicherheit ab. Dennoch besteht grundsätzlich die Gefahr, dass Luftblasen dem Patienten intravenös infundiert werden. In Blutbehandlungsvorrichtungen sind daher zur weiteren Erhöhung der Sicherheit Einrichtungen vorgesehen, mit denen das Auftreten von Luftblasen erkannt werden kann.

Einrichtungen zur Erkennung von Luftblasen im Blut sind beispielsweise aus der DE 102 09 254 A1, DE 10 2005 025 500 B3 und DE 2005 025 515 A1 bekannt. Die bekannten Luftdetektoren beruhen auf der unterschiedlichen Absorption von Ultraschall in flüssigen und gasförmigen Medien sowie der Streuung von Ultraschall an Grenzflächen. Zur Detektion von Luft werden Signalimpulse in das Blut eingekoppelt, während die aus dem Blut austretenden Signalimpulse empfangen werden. Dabei wird auf das Vorhandensein von Luft geschlossen, wenn das Empfangssignal einen Referenzwert unterschreitet. Neben den Ultraschallsensoren sind auch Luftdetektoren bekannt, die auf den unterschiedlichen Dielektrizitätskonstanten und Leitfähigkeiten von flüssigen und gasförmigen Medien beruhen.

Die Entstehung von kleineren oder größeren Luftblasen im Blut kann unterschiedliche Ursachen haben. Mögliche Ursachen für das Eindringen von Luft sind eine Leckage im Schlauchleitungssystem, das Absinken des Blutspiegels in der venösen Tropfkammer oder ein einmaliger Lufteintrag bei einer Infusion eines Arzneimittels in den extrakorporalen Blutkreislauf. Im Blut können aber auch Mikroblasen auftreten, die auf eine Kavitation zurückzuführen sind.

Zum Fördern des Bluts werden im Allgemeinen okkludierende Blutpumpen, insbesondere Rollenpumpen, eingesetzt, die über mehrere Rollen verzügen, mit denen die Schlauchleitung okkludiert wird, woraus sich ein pulsierender Blutfluss ergibt. Eingangsseitig erzeugt die Pumpe in der Blutleitung einen Unterdruck und ausgangsseitig einen Überdruck. Der Unterdruck auf der Saugseite der Pumpe ist auf eine Verengung des Strömungsquerschnitts in der Kanüle zum Anschluss des Patienten zurückzuführen, während der ausgangseitige Überdruck durch den Strömungswiderstand im Schlauchleitungssystem bestimmt wird.

In der Schlauchleitung stromauf der Blutpumpe kann eine Kavitation zur Bildung von Mikroblasen führen. Die Anzahl der Mikroblasen steigt dabei typischerweise von der arteriellen Kanüle in der arteriellen Blutleitung bis zum Ausgang der Blutpumpe an. Der starke Anstieg am Ausgang der Pumpe kann durch die besonders starke Kavitation an der gerade abhebenden Rolle der Rollenpumpe mit einem kurzeitig minimalen Spaltmaß zwischen Rolle und Schlauch und einem hohen Druckgradienten zwischen Pumpenein- und -ausgang erklärt werden. Die einmal entstandenen Mikroblasen sind in dem Schlauchleitungssystem stromab der Pumpe einem Überdruck ausgesetzt, durch den sie sich wieder kontinuierlich auflösen können. Darüber hinaus wird in der Blutbehandlungseinheit, insbesondere im Dialysator, die Anzahl von Mikroblasen durch den Austausch gegen entgaste Dialysierflüssigkeit dadurch verkleinert, dass gelöste Luft aus dem Blutstrom über den Dialysator in das entgaste Dialysat gelangt. Dadurch steigt die Rücklösefähigkeit des Blutes, wodurch die Anzahl der Mikroblasen im Blut stark abnimmt.

Eine gewisse Anzahl von kavitationsbedingten Mikroblasen kann im Schlauchleitungssystem und Dialysator soweit wieder aufgelöst werden, dass sie von den Einrichtungen nicht mehr erkannt wird, mit denen in einem vorgegebenen Zeitintervall die Anzahl der auftretenden Luftblasen mit einem Schwellwert verglichen wird. Eine verstärkte Kavitation führt aber zu einer größeren Anzahl von Luftblasen, die mit den Überwachungseinrichtungen erkannt wird. Wenn während der Behandlung ein bestimmter Schwellwert überschritten wird, löst die Überwachungseinrichtung einen Alarm aus. Da sich zum Zeitpunkt der Schwellwertüberschreitung größere Mengen von Mikroblasen in dem Schlauchabschnitt zwischen der venösen Tropfkammer und der venösen Kanüle befinden, muss die Blutbehandlung zur Abscheidung der Mikroblasen gestoppt werden. Nach dem Stoppen der Dialyse müssen der Patient von der arteriellen und venösen Blutleitung getrennt und die Schlauchenden mit einem speziellen Adapter kurzgeschlossen werden, um das Blut durch den extrakorporalen Blutkreislauf zu pumpen. Während das Blut in dem geschlossenen Kreislauf langsam rezirkuliert, können sich die Mikroblasen auflösen oder können in der venösen Tropfkammer abgeschieden werden. Danach muss der Patient wieder an den extrakorporalen Blutkreislauf angeschlossen werden.

Mit der Überwachung des extrakorporalen Blutkreislaufs wird das Auftreten von Mikroblasen erkannt, ein Alarm ausgelöst und das medizinische Personal zur Einleitung der erforderlichen Maßnahmen aufgefordert, um die Infusion von Mikroblasen auszuschließen. Nachteilig ist jedoch, dass die Unterbrechung der Behandlung für das medizinische Personal mit einem erhöhten Aufwand und für den Patienten mit Unannehmlichkeiten verbunden ist.

Die bei der Entstehung von kavitationsbedingten Luftblasen einzuleitenden Maßnahmen sind von den Maßnahmen zu unterscheiden, die beim Auftreten von Mikroblasen aufgrund von Mikroleckagen im Schlauchleitungssystem oder des Absinkens des Blutspiegels in der Tropfkammer erforderlich sind.

Aus der US 2002/0134134 A1 ist eine Vorrichtung zum Überwachen eines extrakorporalen Blutkreislaufs bekannt, die eine Erkennung eines nicht ordnungsgemäßen Zustandes erlaubt, bei dem die Blutbehandlung unterbrochen wird. Der nicht ordnungsgemäße Zustand wird auf der Grundlage der Erfassung des arteriellen und venösen Drucks mit Drucksensoren und der Größe und Anzahl der Luftblasen mit einem Luftdetektor erkannt. Darüber hinaus wird der arterielle und venöse Druck überwacht, wobei ein Alarm gegeben wird, wenn einer der beiden Drücke unter einen Minimaldruck abfällt. Zusätzlich wird der arterielle Druck überwacht, um sicherzustellen, dass dieser nicht größer als ein Maximaldruck ist.

Der Erfindung liegt die Aufgabe zugrunde, eine Vorrichtung zur Überwachung eines extrakorporalen Blutkreislaufs zu schaffen, die eine Unterscheidung zwischen einer kavitationsbedingten Entstehung von Mikroblasen und einem nicht kavitationsbedingten Lufteintrag erlaubt.

Darüber liegt der Erfindung die Aufgabe zugrunde, ein Verfahren zur Überwachung eines extrakorporalen Blutkreislaufs anzugeben, das eine Unterscheidung zwischen einer kavitationsbedingten und einer nicht kavitationsbedingten Entstehung von Mikroblasen ermöglicht.

Eine Aufgabe der Erfindung ist auch, eine Vorrichtung zur extrakorporalen Blutbehandlung mit einer derartigen Vorrichtung zur Überwachung eines extrakorporalen Blutkreislaufs bereitzustellen.

Die Lösung dieser Aufgaben erfolgt erfindungsgemäß mit den Merkmalen der unabhängigen Patentansprüche, Die abhängigen Ansprüche beziehen sich auf vorteilhafte Ausführungsformen der Erfindung.

Die erfindungsgemäße Vorrichtung und das erfindungsgemäße Verfahren beruhen auf der Überwachung des Unterdrucks im extrakorporalen Blutkreislauf stromauf der Blutpumpe, um zwischen der Entstehung von Mikroblasen, die auf eine Kavitation zurückzuführen sind, und einem Lufteintrag, der nicht durch eine Kavitation bedingt ist, unterscheiden zu können.

In Versuchen hat sich gezeigt, dass eine kavitationsbedingte Entstehung von Luftblasen stromauf der Blutpumpe dann auftreten kann, wenn in dem Abschnitt der Schlauchleitung des extrakorporalen Blutkreislaufs stromauf der Blutpumpe der Betrag des negativen Unterdruck einen bestimmten Grenzwert überschreitet. Dabei wird davon ausgegangen, dass unterhalb dieses Schwellwertes Mikroblasen durch Kavitation nicht erzeugt werden.

Die kritische Schwelle für den gemittelten Unterdruck kann beispielsweise bei 180-190 mmHg liegen.

Die erfindungsgemäße Überwachungsvorrichtung verfügt über eine Einrichtung zum Messen des Unterdrucks im extrakorporalen Blutkreislauf und eine Auswerteinheit, die derart konfiguriert ist, dass auf einen ersten nicht ordnungsgemäßen Zustand geschlossen wird, wenn die Einrichtung zur Erkennung des Auftretens von Luftblasen im extrakorporalen Blutkreislauf Luftblasen erkennt und der gemessene Unterdruck oberhalb eines vorgegebenen Grenzwertes für den Unterdruck liegt, und auf einen zweiten nicht ordnungsgemäßen Zustand geschlossen wird, wenn die Einrichtung zur Erkennung des Auftretens von Luftblasen im extrakorporalen Blutkreislauf Luftblasen erkennt und der gemessene Unterdruck unterhalb des vorgegebenen Grenzwertes für den Unterdruck liegt. Dabei wird davon ausgegangen, dass der erste nicht ordnungsgemäße Zustand, der sich von dem zweiten nicht ordnungsgemäßen Zustand unterscheidet, die kavitationsbedingte Entstehung von Mikroblasen ist. In diesem Zusammenhang wird unter dem Unterdruck die Größe des Betrags des negativen Unterdrucks verstanden, d. h. der Grenzwert für den Unterdruck wird dann überschritten, wenn der gemessene negative Unterdruck oberhalb der kritischen Schwelle liegt.

Wenn der Unterdruck oberhalb des vorgegebenen Grenzwertes liegt und im extrakorporalen Blutkreislauf Mikroblasen erkannt werden, wird darauf geschlossen, dass Mikroblasen durch Kavitation erzeugt werden und die Mechanismen zur Auflösung der Mikroblasen nicht ausreichen, um die Mikroblasen wieder zu entfernen, bevor sie in den Patienten gelangen könnten.

Die erfindungsgemäße Vorrichtung und das erfindungsgemäße Verfahren sehen vor, auf das Risiko einer Mikroblasenbildung schon zu einem Zeitpunkt hinzuweisen, zu dem die Anzahl der in einem Zeitintervall gemessenen Mikroblasen im Allgemeinen noch unter dem Schwellwert liegt, nach dessen Überschreiten zur Unterbrechung der Blutbehandlung aufgefordert wird. Folglich kann das medizinische Personal schon frühzeitig auf das Risiko einer Mikroblasenbildung mit den dazu erforderlichen Maßnahmen reagieren. Beispielsweise kann das medizinische Personal die Blutflussrate verringern, was zu einer Verringerung des Unterdrucks und somit der Mikroblasenbildung führt. Bei der nächsten Dialysebehandlung kann auch eine Kanüle mit einem größeren Querschnitt eingesetzt werden, wodurch höhere Blutflüsse bei niedrigeren Unterdrücken erreicht werden können.

Für den Fall, dass der gemessene Unterdruck unterhalb des vorgegebenen Grenzwertes liegt, wird darauf geschlossen, dass eine kavitationsbedingte Mikroblasenbildung nicht vorliegt. In diesem Fall können beispielsweise Mikroleckagen im arteriellen Schlauchleitungssystem stromauf der Blutpumpe einschließlich der Heparin- oder Infusionsleitungen die Ursache für die Mikroblasenbildung sein. Eine weitere Ursache kann in diesem Fall das Absinken des Blutspiegels in der venösen Tropfkammer sein, so dass das Blut bei regulär gesetzten Pegel nicht unterhalb des Blutspiegels eingeleitet wird, wodurch das Mitreißen von Luft an der Blut-/Luftgrenze vermieden wird. Befindet sich der Bluteinlauf jedoch in der Ebene des Blutspiegels oder sogar höher, wird durch das einströmende Blut systematisch Luft in Form von Mikroblasen in das Blutvolumen der Tropfkammer eingebracht. Dieser Effekt kann auftreten, bevor der Blutspiegelabfall an der Tropfkammer detektiert wird. Die Erkennung einer nicht kavitationsbedingten Mikroblasenbildung kann also nicht nur zur Überprüfung des Schlauchleitungssystems, sondern auch zu dem erforderlichen Spiegelsetzen in der Tropfkammer auffordern.

Eine bevorzugte Ausführungsform der Erfindung sieht vor, dass die in einem vorgegebenen Zeitintervall auftretende Anzahl von Luftblasen ermittelt wird, wobei die Auswerteinheit derart konfiguriert ist, dass auf den ersten nicht ordnungsgemäßen Zustand geschlossen wird, wenn der gemessene Unterdruck oberhalb des vorgegebenen Grenzwertes für den Unterdruck liegt und die Anzahl von Luftblasen in dem vorgegebenen Zeitintervall größer als ein vorgegebener Grenzwert für die Rate der Luftblasen ist und auf den zweiten nicht ordnungsgemäßen Zustand geschlossen wird, wenn der gemessene Unterdruck unterhalb des vorgegebenen Grenzwertes für den Unterdruck liegt und die Anzahl von Luftblasen in dem vorgegebenen Zeitintervall größer als ein vorgegebener Grenzwert für die Rate der Luftblasen ist. Bei dieser Ausführungsform reicht für die Erkennung eines nicht ordnungsgemäßen Zustandes also nicht aus, dass das Auftreten von Mikroblasen erkannt wird, sondern auch eine bestimmte Mikroblasen-Rate überschritten wird, wobei wieder zwischen einer kavitationsbedingten und einer nicht kavitationsbedingten Mikroblasenbildung unterschieden wird.

In Versuchen hat sich weiterhin gezeigt, dass der Grenzwert für den Unterdruck, bei dessen Überschreiten eine kavitationsbedingte Mikroblasenbildung angenommen wird, von dem Patienten abhängig ist. Es hat sich gezeigt, dass der Grenzwert insbesondere von dem Hämatokrit des Bluts abhängt.

Bei einer besonders bevorzugten Ausführungsform ist die Auswerteinheit derart konfiguriert, dass der Grenzwert für den Unterdruck in Abhängigkeit von dem Hämatokrit vorgegeben wird. Dabei wird der Grenzwert für den Unterdruck mit zunehmender Höhe des Hämatokrit verringert. Beispielsweise können für verschiedene Werte des Hämatokrit unterschiedliche Grenzwerte in einem Speicher gespeichert sein, die von der Auswerteinheit aus dem Speicher ausgelesen werden. Der Grenzwert kann aber auch mit einem vorgegebenen mathematischen Zusammenhang berechnet werden. Der Hämatokrit kann wiederum vorgegeben oder gemessen werden. Hierfür kann eine Einrichtung zum Messen des Hämatokrit vorgesehen sein, die ein mit dem Hämatokrit korrelierendes Messsignal erzeugt, dass die Auswerteinheit empfängt.

Wenn auf den ersten nicht ordnungsgemäßen Zustand geschlossen wird und wenn auf den zweiten nicht ordnungsgemäßen Zustand geschlossen wird, erzeugt die Auswerteinheit vorzugsweise ein Steuersignal. Die Auswerteinheit kann für beide nicht ordnungsgemäße Zustände unterschiedliche Steuersignale erzeugen, um zwischen beiden Zuständen unterscheiden zu können.

Bei einer weiteren Ausführungsform ist eine das Steuersignal der Auswerteinheit empfangende Alarmeinheit vorgesehen, die einen akustischen und/oder optischen und/oder taktilen Alarm gibt, wenn die Alarmeinheit ein Steuersignal der Auswerteinheit empfängt.

Die Reduzierung der Blutflussrate zur Verringerung des Risikos der Mikroblasenbildung kann vom Arzt vorgenommen werden. Grundsätzlich ist auch ein automatischer Eingriff in die Blutbehandlung möglich, wenn die Steuereinheit der Blutbehandlungsvorrichtung derart konfiguriert ist, dass die Blutflussrate von einer vorgegebenen ersten Rate auf eine vorgegebene zweite Rate reduziert wird, wenn die Einrichtung zur Erkennung des Auftretens von Luftblasen im extrakorporalen Blutkreislauf Luftblasen erkennt und der gemessene Unterdruck oberhalb des vorgegebenen Grenzwertes für den Unterdruck liegt.

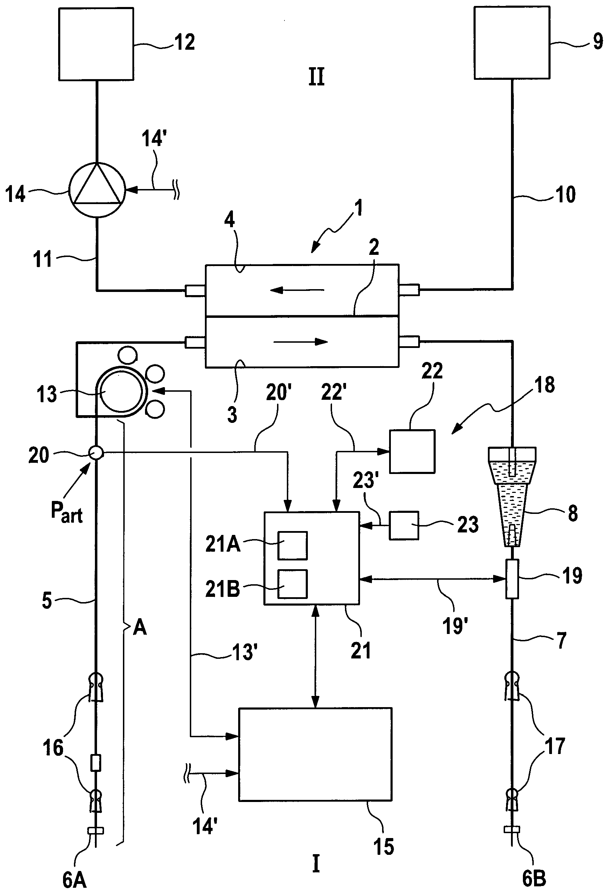

Im Folgenden wird ein Ausführungsbeispiel der Erfindung unter Bezugnahme auf die einzige Figur näher erläutert, die in einer stark vereinfachten schematischen Darstellung, die wesentlichen Komponenten einer Blutbehandlungsvorrichtung zeigt, die über die erfindungsgemäße Überwachungsvorrichtung verfügt.

Die Blutbehandlungsvorrichtung, beispielsweise eine Hämodialysevorrichtung, weist einen Dialysator 1 auf, der durch eine semipermeable Membran 2 in eine Blutkammer 3 und eine Dialysierflüssigkeitskammer 4 getrennt ist. Der Einlass der Blutkammer ist mit dem einen Ende einer arteriellen Blutzuführleitung 5 verbunden, während der Auslass der Blutkammer 3 mit dem einen Ende einer venösen Blutabführleitung 7 verbunden ist, in die eine venöse Tropfkammer 8 geschaltet ist. Die anderen Enden der Blutzuführ- und -abführleitung 5, 7 sind mit einer arteriellen bzw. venösen Kanüle 6A, 6B verbunden. An den Schlauchleitungen sind arterielle und venöse Schlauchklemmen 16, 17 vorgesehen. Die Blutzuführ- und -abfuhrleitung 5, 7 stellen zusammen mit der Blutkammer 3 des Dialysators 1 den extrakorporalen Blutkreislauf I der Hämodialysevorrichtung dar.

Das Dialysierflüssigkeitssystem II der Dialysevorrichtung umfasst eine Dialysierflüssigkeitsquelle 9, von der eine Dialysierflüssigkeitszuführleitung 10 abgeht, die zu der Dialysierflüssigkeitskammer 4 des Dialysators 1 führt. Von der Dialysierflüssigkeitskammer 4 geht eine Dialysierflüssigkeitsabführleitung 11 ab, die zu einem Auslass 12 führt.

In der Blutzuführleitung 5 ist eine okkludierende Blutpumpe 13, insbesondere Rollenpumpe, angeordnet, während in der Dialysierflüssigkeitsabführleitung 11 eine Dialysierflüssigkeitspumpe 14 angeordnet ist. Blutpumpe 13 und Dialysierflüssigkeitspumpe 14 fördern während der Blutbehandlung Blut im extrakorporalen Blutkreislauf I bzw. Dialysierflüssigkeit im Dialysierflüssigkeitssystem II.

Die Hämodialysevorrichtung umfasst eine zentrale Steuereinheit 15, die über Steuerleitungen 13', 14' mit der Blutpumpe 13 bzw. Dialysierflüssigkeitspumpe 14 verbunden ist. Die Steuereinheit 15 gibt für die Blut- und Dialysierflüssigkeitspumpe eine bestimmte Blutfluss- oder Dialysierflüssigkeitsrate vor.

Darüber hinaus verfügt die Hämodialysevorrichtung über eine Vorrichtung 18 zum Überwachen des extrakorporalen Blutkreislaufs I, die bei dem vorliegenden Ausführungsbeispiel Bestandteil der Hämodialysevorrichtung ist, aber auch eine separate Baugruppe bilden kann.

Die Überwachungsvorrichtung 18 weist eine Einrichtung 19 zur Erkennung des Auftretens von Mikroblasen auf, die als solche zum Stand der Technik gehört. Derartige Einrichtungen sind beispielsweise in der DE 10 2005 025 515 A1 oder DE 10 2005 025 500 B3 beschrieben. Diese Einrichtung 19, die einen Ultraschall-Sender und einen Ultraschall-Empfänger aufweist, die stromab der Tropfkammer 8 an der venösen Blutleitung 7 angeordnet sind, ermittelt die in einem vorgegebenen Zeitintervall im Blut stromab der Tropfkammer 8 auftretenden Mikroblasen, d. h. die Mikroblasenrate. Darüber hinaus weist die Überwachungseinrichtung 18 eine Einrichtung 20 zum Messen des Unterdrucks Pₐᵣₜ in dem Abschnitt A der arteriellen Schlauchleitung 5 stromauf der Blutpumpe 13 auf. Beide Einrichtungen 19, 20 sind über Datenleitungen 19', 20' mit einer Auswerteinheit 21 der Überwachungsvorrichtung 18 verbunden. Mit Ausnahme der Sensorik, d.h. dem Ultraschall-Sender und -Empfänger sowie dem Drucksensor, können sämtliche Komponenten der Überwachungsvorrichtung 18 Bestandteil einer zentralen Recheneinheit (Mikroprozessor) sein, die wiederum Bestandteil der zentralen Steuereinheit 15 der Blutbehandlungsvorrichtung sein kann, wobei auf der Steuereinheit ein Programm (Software) läuft, nach dem die einzelnen Verfahrensschritte ausgeführt werden. An die Auswerteinheit 21 ist über eine weitere Datenleitung 22' eine Alarmeinheit 22 angeschlossen, die einen akustischen und/oder optischen und/oder taktilen Alarm gibt, wenn sie von der Auswerteinheit 21 ein Alarmsignal empfängt.

Die Auswerteinheit 21 weist einen ersten und einen zweiten Komparator 21A, 21B auf. Der erste Komparator 21A vergleicht die ermittelte Anzahl von Mikroblasen in einem vorgegebenen Zeitintervall mit einem vorgegebenen ersten Grenzwert für die Mikroblasenrate, während der zweite Komparator 21B den Betrag des gemessenen negativen Unterdrucks Pₐᵣₜ in dem arteriellen Schlauchleitungsabschnitt A stromauf der Blutpumpe 13 mit einem vorgegebenen zweiten Grenzwert vergleicht, der eine kritische Schwelle für das Auftreten von kavitationsbedingten Mikroblasen darstellt. Der zweite Grenzwert liegt beispielsweise bei 180 - 190 mmHg. Wenn die Mikroblasenrate oberhalb des ersten Grenzwerts und der Betrag des Unterdrucks über dem zweiten Grenzwert liegen, erzeugt die Auswerteinheit 21 ein Steuersignal und ein erstes Alarmsignal. Die Alarmeinheit gibt dann einen ersten Alarm, der signalisiert, dass eine kavitationsbedingte Mikroblasenbildung vorliegt. Daraufhin können die hierzu erforderlich Gegenmaßnahmen eingeleitet werden. Beispielsweise kann die Blutflussarte reduziert werden. Bei dem vorliegenden Ausführungsbeispiel kann die Steuereinheit 21 auch eine automatische Reduzierung der Blutflussrate von einem für die Blutbehandlung zuvor eingestellten Wert auf einen niedrigeren Wert vorsehen, wenn die Steuereinheit das Steuersignal der Auswerteinheit 21 empfängt.

Die Auswerteinheit 21 erzeugt ein zweites Alarmsignal, wenn die Mikroblasenrate oberhalb des ersten Grenzwerts und der Betrag des Unterdrucks unter dem zweiten Grenzwert liegen. Dann liegt keine kavitationsbedingte Mikroblasenbildung vor, so dass andere Gegenmaßnahmen getroffen werden können.

Es hat sich in Versuchen gezeigt, dass das Entgasungsphänomen, das nur bei einem relativ großen Unterdruck auftreten dürfte, auch vom Hämatokrit des Bluts abhängt. Bei gleichem arteriellen Unterdruck wurden bei hohem Hämatokrit höhere Mikroblasenraten gemessen, während bei kleinem Hämatokrit kleinere Mikroblasenraten gemessen wurden. Die Überwachungsvorrichtung 18 kann auch eine Einrichtung 23 zum Messen des Hämatokrit aufweisen, die ebenfalls nur andeutungsweise dargestellt ist. Diese Einrichtung 23 erzeugt ein mit dem Hämatokrit korrelierendes Messsignal, das die Auswerteinheit 21 über eine Datenleitung 23' empfängt, wobei die Auswerteinheit derart konfiguriert ist, dass sie den zweiten Grenzwert in Abhängigkeit von dem Messsignal vorgibt. Dabei gibt die Auswerteinheit einen niedrigeren Grenzwert bei einem höheren Hämatokrit und einen höheren Grenzwert bei einem niedrigeren Hämatokrit vor.

## Patentansprüche

1. Vorrichtung zum Überwachen eines extrakorporalen Blutkreislaufs, in dem eine Blutpumpe zum Fördem von Blut angeordnet ist, mit einer Einrichtung (19) zur Erkennung des Auftretens von Luftblasen im Blut, das im extrakorporalen Blutkreislauf strömt, einer Einrichtung (20) zum Messen des Unterdrucks (Pₐᵣₜ) im extrakorporalen Blutkreislauf stromauf der Blutpumpe und einer Auswerteinheit (21),
**dadurch gekennzeichnet,**
**dass** die Auswerteinheit (21) derart konfiguriert ist, dass auf einen ersten nicht ordnungsgemäßen Zustand geschlossen wird, wenn die Einrichtung (19) zur Erkennung des Auftretens von Luftblasen im extrakorporalen Blutkreislauf Luftblasen erkennt und der gemessene Unterdruck (Pₐᵣₜ) oberhalb eines vorgegebenen Grenzwertes für den Unterdruck liegt, und auf einen zweiten nicht ordnungsgemäßen Zustand geschlossen wird, wenn die Einrichtung (19) zur Erkennung des Auftretens von Luftblasen im extrakorporalen Blutkreislauf Luftblasen erkennt und der gemessene Unterdruck unterhalb des vorgegebenen Grenzwertes für den Unterdruck liegt, wobei sich der erste nicht ordnungsgemäße Zustand von dem zweiten nicht ordnungsgemäßen Zustand unterscheidet.

2. Vorrichtung nach Anspruch 1, **dadurch gekennzeichnet, dass** die Einrichtung (19) zur Erkennung des Auftretens von Luftblasen derart konfiguriert ist, dass die in einem vorgegebenen Zeitintervall auftretende Anzahl von Luftblasen ermittelt wird, wobei die Auswerteinheit (21) derart konfiguriert ist, dass auf den ersten nicht ordnungsgemäßen Zustand geschlossen wird, wenn der gemessene Unterdruck oberhalb des vorgegebenen Grenzwertes für den Unterdruck liegt und die Anzahl von Luftblasen in dem vorgegebenen Zeitintervall größer als ein vorgegebener Grenzwert für die Rate der Luftblasen ist und auf den zweiten nicht ordnungsgemäßen Zustand geschlossen wird, wenn der gemessene Unterdruck unterhalb des vorgegebenen Grenzwertes für den Unterdruck liegt und die Anzahl von Luftblasen in dem vorgegebenen Zeitintervall größer als ein vorgegebener Grenzwert für die Rate der Luftblasen ist.

3. Vorrichtung nach Anspruch 2, **dadurch gekennzeichnet, dass** die Auswerteinheit (21) derart konfiguriert ist, dass der Grenzwert für den Unterdruck in Abhängigkeit von dem Hämatokrit vorgegeben wird.

4. Vorrichtung nach Anspruch 3, **dadurch gekennzeichnet, dass** die Auswerteinheit (21) derart konfiguriert ist, dass mit zunehmender Höhe des Hämatokrit der Grenzwert für den Unterdruck verringert wird.

5. Vorrichtung nach Anspruch 3 oder 4, **dadurch gekennzeichnet, dass** die Vorrichtung (18) zum Überwachen des extrakorporalen Blutkreislaufs eine Einrichtung (23) zum Messen des Hämatokrit aufweist, die ein mit dem Hämatokrit korrelierendes Messsignal erzeugt, das die Auswerteinheit empfängt.

6. Vorrichtung nach einem der Ansprüche 1 bis 5, **dadurch gekennzeichnet, dass** die Auswerteinheit (21) ein Steuersignal erzeugt, wenn auf den ersten und wenn auf den zweiten nicht ordnungsgemäßen Zustand geschlossen wird.

7. Vorrichtung nach Anspruch 6, **dadurch gekennzeichnet, dass** eine das Steuersignal der Auswerteinheit (21) empfangende Alarmeinheit (22) vorgesehen ist, die einen akustischen und/oder optischen und/oder taktilen Alarm gibt, wenn die Alarmeinheit das Steuersignal der Auswerteinheit empfängt.

8. Vorrichtung zur extrakorporalen Blutbehandlung mit einem extrakorporalen Blutkreislauf (I) und einer Vorrichtung (18) nach einem der Ansprüche 1 bis 7.

9. Vorrichtung nach Anspruch 8, **dadurch gekennzeichnet, dass** der extrakorporale Blutkreislauf (I) eine Blutbehandlungseinheit (1) und eine zu der Blutbehandlungseinheit führende arterielle Blutleitung (5) und eine von der Blutbehandlungseinheit abgehende venöse Blutleitung (7) aufweist, wobei die Blutpumpe (13) in der arteriellen Blutleitung (5) angeordnet ist.

10. Vorrichtung nach Anspruch 8 oder 9, **dadurch gekennzeichnet, dass** die Blutbehandlungsvorrichtung eine Steuereinheit (15) zum Ansteuern der Blutpumpe (13) mit einer vorgegebenen Blutflussrate aufweist, wobei die Steuereinheit (15) derart konfiguriert ist, dass die Blutflussrate von einer vorgegebenen ersten Rate auf eine vorgegebene zweite Rate reduziert wird, wenn die Einrichtung (19) zur Erkennung des Auftretens von Luftblasen im extrakorporalen Blutkreislauf Luftblasen erkennt und der gemessene Unterdruck (Pₐᵣₜ) oberhalb des vorgegebenen Grenzwertes für den Unterdruck liegt.

11. Verfahren zum Überwachen eines extrakorporalen Blutkreislaufs, in dem eine Blutpumpe zum Fördern von Blut angeordnet ist, bei dem das Auftreten von Luftblasen im Blut, das im extrakorporalen Blutkreislauf strömt, erkannt wird, wobei der Unterdruck (Pₐᵣₜ) im extrakorporalen Blutkreislauf stromauf der Blutpumpe gemessen wird, **dadurch gekennzeichnet, dass** auf einen ersten nicht ordnungsgemäßen Zustand geschlossen wird, wenn das Auftreten von Luftblasen im extrakorporalen Blutkreislauf erkannt wird und der gemessene Unterdruck oberhalb eines vorgegebenen Grenzwertes für den Unterdruck liegt, und auf einen zweiten nicht ordnungsgemäßen Zustand geschlossen wird, wenn das Auftreten von Luftblasen im extrakorporalen Blutkreislauf erkannt wird und der gemessene Unterdruck unterhalb des vorgegebenen Grenzwertes für den Unterdruck liegt, wobei sich der erste nicht ordnungsgemäße Zustand von dem zweiten nicht ordnungsgemäßen Zustand unterscheidet.

12. Verfahren nach Anspruch 11, **dadurch gekennzeichnet, dass** die in einem vorgegebenen Zeitintervall auftretende Anzahl von Luftblasen ermittelt wird, wobei auf den ersten nicht ordnungsgemäßen Zustand geschlossen wird, wenn der gemessene Unterdruck oberhalb des vorgegebenen Grenzwertes für den Unterdruck liegt und die Anzahl von Luftblasen in dem vorgegebenen Zeitintervall größer als ein vorgegebener Grenzwert für die Rate der Luftblasen ist und auf den zweiten nicht ordnungsgemäßen Zustand geschlossen wird, wenn der gemessene Unterdruck unterhalb des vorgegebenen Grenzwertes für den Unterdruck liegt und die Anzahl von Luftblasen in dem vorgegebenen Zeitintervall größer als ein vorgegebener Grenzwert für die Rate der Luftblasen ist.

13. Verfahren nach Anspruch 12, **dadurch gekennzeichnet, dass** der Grenzwert für den Unterdruck in Abhängigkeit von dem Hämatokrit vorgegeben wird.

14. Verfahren nach Anspruch 13, **dadurch gekennzeichnet, dass** mit zunehmender Höhe des Hämatokrit der Grenzwert für den Unterdruck verringert wird.

15. Verfahren nach Anspruch 13 oder 14, **dadurch gekennzeichnet, dass** der Hämatokrit während der Blutbehandlung gemessen wird.

16. Verfahren nach einem der Ansprüche 11 bis 15, **dadurch gekennzeichnet, dass** ein akustischer und/oder optischer und/oder taktiler Alarm gegeben wird, wenn der erste und wenn der zweite nicht ordnungsgemäße Zustand erkannt wird.

## Claims

1. Device for monitoring an extracorporeal blood circuit in which a blood pump is disposed to convey blood, comprising an apparatus (19) for detecting the occurrence of air bubbles in blood that flows in the extracorporeal blood circuit, an apparatus (20) for measurement of the negative pressure (Pₐᵣₜ) in the extracorporeal blood circuit upstream of the blood pump and a processing unit (21),
**characterised in**
**that** the processing unit (21) is configured so that, on a first defective condition is deduced when the apparatus (19) for detecting the occurrence of air bubbles in the extracorporeal blood circuit detects air bubbles and the measured negative pressure (Pₐᵣₜ) is above a predetermined limit value for the negative pressure, and a second defective condition is deduced when the apparatus (19) for detection of the occurrence of air bubbles in the extracorporeal blood circuit detects air bubbles and the measured negative pressure is below the predetermined limit value for the negative pressure, wherein the first defective condition differs from the second defective condition.

2. Device in accordance with claim 1, **characterised in that** the apparatus (19) for detecting the occurrence of air bubbles is configured so that the number of air bubbles occurring in a predetermined time interval is determined, wherein the processing unit (21) is configured so that, the first defective condition is deduced when the measured negative pressure is above the predetermined limit value for the negative pressure and the number of air bubbles in the predetermined time interval is greater than a predetermined limit value for the rate of air bubbles, and the second defective condition is deduced when the measured negative pressure is below the predetermined limit value for the negative pressure and the number of air bubbles in the predetermined time interval is greater than a predetermined limit value for the rate of air bubbles.

3. Device in accordance with claim 2, **characterised in that** the processing unit (21) is configured so that the limit value for the negative pressure is specified as a function of the haematocrit.

4. Device in accordance with claim 3, **characterised in that** the processing unit (21) is configured so that the limit value for the negative pressure is reduced with increasing level of the haematocrit.

5. Device in accordance with claim 3 or 4, **characterised in that** the device (18) for monitoring the extracorporeal blood circuit comprises an apparatus (23) for measuring the haematocrit, which generates a measurement signal correlated to the haematocrit which is received by the processing unit.

6. Device in accordance with one of the claims 1 to 5, **characterised in that** the processing unit (21) generates a control signal when the first and the second defective condition is deduced.

7. Device in accordance with claim 6, **characterised in that** an alarm unit (22) is provided, which receives the control signal of the processing unit (21) and gives an acoustic and/or optical and/or tactile alarm when the alarm unit receives the control signal of the processing unit.

8. Device for extracorporeal blood treatment with an extracorporeal blood circuit (I) and a device (18) in accordance with one of the claims 1 to 7.

9. Device in accordance with claim 8, **characterised in that** the extracorporeal blood circuit (I) comprises a blood treatment unit (1) and an arterial blood line (5) leading to the blood treatment unit and a venous blood line (7) leading from the blood treatment unit, wherein the blood pump (13) is positioned in the arterial blood line (5).

10. Device in accordance with claim 8 or 9, **characterised in that** the blood treatment device has a control unit (15) for actuating the blood pump (13) with a predetermined blood flow rate, wherein the control unit (15) is configured so that the blood flow rate is reduced from a predetermined first rate to a predetermined second rate when the apparatus (19) for detecting the occurrence of air bubbles in the extracorporeal blood circuit detects air bubbles and the measured negative pressure (Pₐᵣₜ) is above the predetermined limit value for the negative pressure.

11. Method for monitoring an extracorporeal blood circuit, in which a blood pump is disposed to convey blood, with which the occurrence of air bubbles in the blood that flows in the extracorporeal blood circuit is detected, wherein the negative pressure (Pₐᵣₜ) in the extracorporeal blood circuit is measured upstream of the blood pump, **characterised in that** a first defective condition is deduced when the occurrence of bubbles is detected in the extracorporeal blood circuit and the measured negative pressure is above a predetermined limit value for the negative pressure, and a second defective condition is deduced when the occurrence of air bubbles is detected in the extracorporeal blood circuit and the measured negative pressure is below the predetermined limit value for the negative pressure, wherein the first defective condition differs from the second defective condition.

12. Method in accordance with claim 11, **characterised in that** the number of air bubbles occurring in a predetermined time interval is determined, wherein the first defective condition is deduced when the measured negative pressure is above the predetermined limit value for the negative pressure and the number of air bubbles in the predetermined time interval is greater than a predetermined limit value for the rate of air bubbles, and the second defective condition is deduced when the measured negative pressure is below the predetermined limit value for the negative pressure and the number of air bubbles in the predetermined time interval is greater than a predetermined limit value for the rate of air bubbles.

13. Method in accordance with claim 12, **characterised in that** the limit value for the negative pressure is specified as a function of the haematocrit.

14. Method in accordance with claim 13, **characterised in that** the limit value for the negative pressure is reduced with increasing level of the haematocrit.

15. Method in accordance with claim 13 or 14, **characterised in that** the haematocrit is measured during the blood treatment.

16. Method in accordance with one of the claims 11 to 15, **characterised in that** an acoustic and/or optical and/or tactile alarm is given when the first and the second defective condition is detected.

## Revendications

1. Dispositif de surveillance d'un circuit sanguin extracorporel, dans lequel une pompe à sang est agencée pour transporter du sang, avec un équipement (19) de détection de l'apparition de bulles d'air dans le sang, qui s'écoule dans le circuit sanguin extracorporel, un équipement (20) de mesure de la sous-pression (Pₐᵣₜ) dans le circuit sanguin extracorporel en amont de la pompe à sang et une unité d'évaluation (21),
**caractérisé en ce**
**que** l'unité d'évaluation (21) est configurée de telle sorte qu'il est conclu à un premier état non conforme, lorsque l'équipement (19) de détection de l'apparition de bulles d'air détecte des bulles d'air dans le circuit sanguin extracorporel et la sous-pression (Pₐᵣₜ) mesurée est supérieure à une valeur limite prédéfinie pour la sous-pression, et qu'il est conclu à un deuxième état non conforme, lorsque l'équipement (19) de détection de l'apparition de bulles d'air détecte des bulles d'air dans le circuit sanguin extracorporel et la sous-pression mesurée est inférieure à la valeur limite prédéfinie pour la sous-pression, dans lequel le premier état non conforme se distingue du deuxième état non conforme.

2. Dispositif selon la revendication 1, **caractérisé en ce que** l'équipement (19) de détection de l'apparition de bulles d'air est configuré de telle sorte que le nombre de bulles d'air apparaissant dans un intervalle de temps prédéfini est déterminé, dans lequel l'unité d'évaluation (21) est configurée de telle sorte qu'il est conclu au premier état non conforme, lorsque la sous-pression mesurée est supérieure à la valeur limite prédéfinie pour la sous-pression et le nombre de bulles d'air dans l'intervalle de temps prédéfini est supérieur à une valeur limite prédéfinie pour le taux de bulles d'air et qu'il est conclu au deuxième état non conforme, lorsque la sous-pression mesurée est inférieure à la valeur limite prédéfinie pour la sous-pression et le nombre de bulles d'air dans l'intervalle de temps prédéfini est supérieur à une valeur limite prédéfinie pour le taux de bulles d'air.

3. Dispositif selon la revendication 2, **caractérisé en ce que** l'unité d'évaluation (21) est configurée de telle sorte que la valeur limite pour la sous-pression est prédéfinie en fonction de l'hématocrite.

4. Dispositif selon la revendication 3, **caractérisé en ce que** l'unité d'évaluation (21) est configurée de telle sorte que la valeur limite pour la sous-pression est diminuée à mesure que le niveau d'hématocrite augmente.

5. Dispositif selon la revendication 3 ou 4, **caractérisé en ce que** le dispositif (18) de surveillance du circuit sanguin extracorporel présente un équipement (23) de mesure de l'hématocrite, qui génère un signal de mesure corrélant avec l'hématocrite, que l'unité d'évaluation reçoit.

6. Dispositif selon l'une quelconque des revendications 1 à 5, **caractérisé en ce que** l'unité d'évaluation (21) génère un signal de commande, lorsqu'il est conclu au premier état non conforme et lorsqu'il est conclu au deuxième état non conforme.

7. Dispositif selon la revendication 6, **caractérisé en ce qu'**une unité d'alarme (22) recevant le signal de commande de l'unité d'évaluation (21) est prévue, qui émet une alarme acoustique et/ou optique et/ou tactile, lorsque l'unité d'alarme reçoit le signal de commande de l'unité d'évaluation.

8. Dispositif de traitement du sang extracorporel avec un circuit sanguin extracorporel (I) et un dispositif (18) selon l'une quelconque des revendications 1 à 7.

9. Dispositif selon la revendication 8, **caractérisé en ce que** le circuit sanguin extracorporel (I) présente une unité de traitement du sang (1) et une conduite de sang artériel (5) conduisant à l'unité de traitement du sang et une conduite de sang veineux (7) partant de l'unité de traitement du sang, dans lequel la pompe à sang (13) est agencée dans la conduite de sang artériel (5).

10. Dispositif selon la revendication 8 ou 9, **caractérisé en ce que** le dispositif de traitement du sang présente une unité de commande (15) pour la commande de la pompe à sang (13) avec un débit sanguin prédéfini, dans lequel l'unité de commande (15) est configurée de telle sorte que le débit sanguin est réduit d'un premier taux prédéfini à un deuxième taux prédéfini, lorsque l'équipement (19) de détection de l'apparition de bulles de sang détecte des bulles d'air dans le circuit sanguin extracorporel et la sous-pression (Pₐᵣₜ) mesurée est supérieure à la valeur limite prédéfinie pour la sous-pression.

11. Procédé de surveillance d'un circuit sanguin extracorporel, dans lequel une pompe à sang est agencée pour transporter du sang, dans lequel l'apparition de bulles d'air dans le sang, qui s'écoule dans le circuit sanguin extracorporel, est détectée, dans lequel la sous-pression (Pₐᵣₜ) dans le circuit sanguin extracorporel est mesurée en amont de la pompe à sang, **caractérisé en ce qu'**il est conclu à un premier état non conforme, lorsque l'apparition de bulles d'air est détectée dans le circuit sanguin extracorporel et la sous-pression mesurée est supérieure à une valeur limite prédéfinie pour la sous-pression, et qu'il est conclu à un deuxième état non conforme, lorsque l'apparition de bulles d'air est détectée dans le circuit sanguin extracorporel et la sous-pression mesurée est inférieure à la valeur limite prédéfinie pour la sous-pression, dans lequel le premier état non conforme se distingue du deuxième état non conforme.

12. Procédé selon la revendication 11, **caractérisé en ce que** le nombre de bulles d'air apparaissant dans un intervalle de temps prédéfini est déterminé, dans lequel il est conclu au premier état non conforme, lorsque la sous-pression mesurée est supérieure à la valeur limite prédéfinie pour la sous-pression et le nombre de bulles d'air dans l'intervalle de temps prédéfini est supérieur à une valeur limite prédéfinie pour le taux de bulles d'air et il est conclu au deuxième état non conforme, lorsque la sous-pression mesurée est inférieure à la valeur limite prédéfinie pour la sous-pression et le nombre de bulles d'air dans l'intervalle de temps prédéfini est supérieur à une valeur limite prédéfinie pour le taux de bulles d'air.

13. Procédé selon la revendication 12, **caractérisé en ce que** la valeur limite pour la sous-pression est prédéfinie en fonction de l'hématocrite.

14. Procédé selon la revendication 13, **caractérisé en ce que** la valeur limite pour la sous-pression est diminuée à mesure que le niveau d'hématocrite augmente.

15. Procédé selon la revendication 13 ou 14, **caractérisé en ce que** l'hématocrite est mesuré pendant le traitement du sang.

16. Dispositif selon l'une quelconque des revendications 11 à 15, **caractérisé en ce qu'**une alarme acoustique et/ou optique et/ou tactile est émise, lorsque le premier état non conforme est détecté et lorsque le deuxième état non conforme est détecté.
